# EUROPEAN PATENT SPECIFICATION

(11) **EP 2 874 603 B1**
(45) Date of publication and mention of the grant of the patent: **24.10.2018**
(21) Application number: 13765500.7
(22) Date of filing: 15.07.2013
(51) Int. Cl.: A61K 9/00, A61K 47/02, A61K 47/26, A61P 7/08

(54) **GLUCOSALINE MAINTENANCE SOLUTION FOR PEDIATRIC PARENTERAL ADMINISTRATION**
GLUCOSALINPFLEGELÖSUNG ZUR PARENTERALEN VERABREICHUNG BEI KINDERN
SOLUTION DE MAINTENANCE GLUCOSALINE DESTINÉE À UNE ADMINISTRATION PARENTÉRALE PÉDIATRIQUE

(30) Priority: 17.07.2012 IT RM20120341
(43) Date of publication of application: 27.05.2015
(73) Proprietor: Istituto di Ricovero E Cura A Carattere Scientifico Materno-Infantile Burlo Garofolo- Ospedale di Alta Specializzazione E di Rilievo, 34137 Trieste (IT)
(72) Inventor: CALLIGARIS, Lorenzo, 34137 Trieste (TS) (IT); GERMANI, Claudio, 34137 Trieste (TS) (IT); ZANON, Davide, 34137 Trieste (TS) (IT)
(74) Representative: Spadaro, Marco
(86) International application number: PCT/IB2013/055827
(87) International publication number: WO 2014/013423

(56) References cited:
- "Intravenous Potassium Guideline", , 1 March 2011 (2011-03-01), pages 1-21, XP055057560, Retrieved from the Internet: URL:http://www.formulary.cht.nhs.uk/pdf,_d oc_files_etc/MMC/118_-_IV_Potassium_Guidel ine_-_inc_post_MMC_update.pdf [retrieved on 2013-03-22]
- ARITOMI S ET AL: "Infusion solution for intravenous administration, comprising amino acid, and has preset osmotic pressure ratio with respect to physiological saline, non-protein calorie per total nitrogen and total energy", WPI / THOMSON,, vol. 2005, no. 38, 26 May 2005 (2005-05-26), XP002693935, -& JP 2005 132831 A (AJINOMOTO KK) 26 May 2005 (2005-05-26)
- SHIGETA M: "Infusion solution for peripheral intravenous administration, comprises amino acid, saccharide and electrolyte accommodated in multi-chambered container, where amino acid solution is accommodated in first chamber", WPI / THOMSON,, vol. 2007, no. 44, 7 June 2007 (2007-06-07), XP002693936, -& JP 2007 137836 A (AJINOMOTO KK) 7 June 2007 (2007-06-07)
- GERBEN KEIJZERS ET AL: "Survey of paediatric intravenous fluid prescription: Are we safe in what we know and what we do?", EMERGENCY MEDICINE AUSTRALASIA, vol. 24, no. 1, 24 October 2011 (2011-10-24), pages 86-97, XP055056773, ISSN: 1742-6731, DOI: 10.1111/j.1742-6723.2011.01503.x cited in the application
- THOMAS G SABA ET AL: "A randomized controlled trial of isotonic versus hypotonic maintenance intravenous fluids in hospitalized children", BMC PEDIATRICS, BIOMED CENTRAL, LONDON, GB, vol. 11, no. 1, 23 September 2011 (2011-09-23), page 82, XP021110277, ISSN: 1471-2431, DOI: 10.1186/1471-2431-11-82

## Description

### Background of the Invention

The present invention relates to the pharmaceutical field, in particular to a solution for electrolyte replacement, suitable for parenteral administration and pediatric use. The solution is particularly useful in hospital contexts for the maintenance of the hydro-electrolyte balance, in particular for preventing iatrogenic hyponatremia. Parenteral maintenance of hydration in pediatric patients is usually carried out using electrolyte maintenance solutions. Since the parenteral sodium and potassium supply provided by standard electrolyte maintenance solutions can be insufficient in the child, and the deficiency of these solutes can lead to secondary hyponatremia along with potentially very severe, sometimes lethal, consequences, a solution for pediatric use, and parenteral administration has been developed, which allows to provide a proper supply of potassium and sodium for replacing electrolytes and, at the same time, maintaining proper glucose levels, without inducing acidosis.

### Prior art

EP 0393909 discloses a composition for treating diarrhea comprising loperamide, or a salt or a prodrug thereof, and an oral rehydrating agent, along with appropriate excipients. The rehydrating agent consists of sugars, salts and amino acids, wherein sugars can be sucrose, fructose or glucose, salts are sodium, potassium, calcium or magnesium salts and amino acids are alanine or glycine. In the composition, loperamide is in an amount of 0.5-10 mg, preferably 1-4 mg per unit dose; the hydrating agent is in an amount of 1-10 g, wherein glucose is 1-6 g, wherein salts are 0.1-4 g per unit dose. The unit dose is established based on a subject of 70 kg average weight. The composition is for oral administration, preferably for reconstitutable formulations.

US4942042 discloses a composition for treating diarrhea comprising an absorbent material able to absorb pathogenic intestinal bacteria (smectite), sodium salts, potassium salts and a sugar. Salts are NaCl, KCI and NaH-CO₃ and the sugar is glucose or dextrose. This document reports clinical data related to children, from 1 to 24 months old, hospitalized with diarrhea needing of oral rehydration.

EP0482580 discloses a composition for treating diarrhea comprising loperamide hydrochloride and a saccharide, monosaccharide, or oligosaccharide or a polyalcohol-sugar in an amount at least 3000-fold lower than loperamide.

EP0919239 discloses the treatment of acute gastroenteritis by administering a rehydration solution comprising at least a compound having a synergistic action selected from antiseptic, prebiotic, bowel transit modifier, and clay. Dehydration in children, due to acute gastroenteritis, is treated by oral administration of a solution specific for dehydration comprising glucose, sucrose, citrate, chloride, potassium, and sodium ions, and at least a compound selected from antiseptic, prebiotic, bowel transit modifier, and clay.

FR255806 discloses a solution for orally rehydration of infants with gastroenteritis, mainly virus-derived, comprising citrate, chloride, potassium, and sodium.

EP0204601 discloses a composition for treating diarrhea comprising: clay based on double silicate of aluminium and magnesium, which absorbs pathogenic intestinal bacteria, sodium salts, potassium salts, and sugar such as glucose or dextrose, and preferably a bicarbonate, furthermore it can comprise an agent which aids to dispersing the absorbent substances in water.

WO2004032823 discloses a composition suitable for minimizing gastroenteritis side effects in adults, such as discomfort and fatigue. The solution comprises an intestinal antiseptic, an agent which alters the bowel transit, and glucose and/or sucrose, the latter are in an amount such that osmolarity is comprised between 260 and 290 mosm, in order to enhance sodium absorption.

In pediatric patients, parenteral hydration maintenance is usually carried out using electrolyte maintenance solutions. Said solutions have low contents of sodium and potassium, which are inadequate for pediatric use (Keijzers G, McGrath M, Bell C. Survey of paediatric intravenous fluid prescription: are we safe in what we know and what we do? Emerg Med Australas. 2012 Feb;24(1):86-97).

Furthermore, the scientific literature reports the well-established use of isotonic solutions for maintenance hydration of the hospitalized child under stress conditions, pain or respiratory infection, wherein a hyponatremia is likely to occur due to the concurrent presence of syndrome caused by improper secretion of antidiuretic hormone (Alves JT, Troster EJ, de Oliveira CA. Isotonic saline solution as maintenance intravenous fluid therapy to prevent acquired hyponatremia in hospitalized children. J Pediatr (Rio J). 2011 Nov-Dec;87(6):478-86; Saba TG, Fairbairn J, Houghton F, Laforte D, Foster BJ.A randomized controlled trial of isotonic versus hypotonic maintenance intravenous fluids in hospitalized children. BMC Pediatr. 2011 Sep 23; 11:82; Choong K, Arora S, Cheng J, Farrokhyar F, Reddy D, Thabane L, Walton JM. Hypotonic versus isotonic maintenance fluids after surgery for children: a randomized controlled trial. Pediatrics. 2011 Nov;128(5):857-66. Epub 2011 Oct 17; Beck CE. Hypotonic versus isotonic maintenance intravenous fluid therapy in hospitalized children: a systematic review. Clin Pediatr (Phila). 2007 Nov;46(9):764-70;Hoorn EJ, Geary D, Robb M, Halperin ML, Bohn D. Acute hyponatremia related to intravenous fluid administration in hospitalized children: an observational study. Pediatrics. 2004 May;113(5):1279-84).

In addition, pediatric maintenance hydration can be performed through non-isotonic solutions or balanced maintenance hydro-electrolyte solutions, or Ringer lactate, or by extemporaneous combinations of solutions comprising, for example, one-third of physiological solution, and 2/3 of hydro-electrolyte maintenance solution, or 50% hydro-electrolyte maintenance solution, and 50% physiological solution. Said solutions do not allow a proper supply of electrolytes and/or sugars.

The use of Using Ringer lactate solutions is known, nevertheless they have a restricted use in pediatrics such as, for example, in anesthesia procedures in post-surgery, but their use is unsuitable since the potassium amount is insufficient (4 mEq/L).

In fact, without specific preparations, in the actual clinical practice the parenteral hydration maintenance in pediatric patients is carried out by electrolyte maintenance solutions, which have low contents of sodium and potassium, insufficient glucose supply and too low osmolarity.

In children, the insufficient supply of sodium and potassium provided by standard electrolyte maintenance solutions leads to very severe, sometimes lethal, consequences since the deficiency of said solutes can cause a secondary hyponatremia in children.

On the contrary, the physiological solution has a sufficiently high osmolarity compared with standard electrolyte maintenance solutions, but the pediatrician's reluctance in using it as isotonic solution, instead of hypo-osmolar solutions, arises from some limitations thereof: absence of potassium, absence of glucose, risk of expansion of the extracellular volume (Holliday MA. Isotonic saline expands extracellular fluid and is inappropriate for maintenance therapy. Pediatrics. 2005 Jan;115(1):193-4; author reply 194), hyperchloraemia and acidosis risk (Mann C, Held U, Herzog S, Baenziger O. Impact of normal saline infusion on postoperative metabolic acidosis Paediatr Anaesth. 2009 Nov;19(11):1070-7).

Using extemporaneous freshly made combinations of solutions (saline 50%-diluted with 10% glucosate solution and supplemented with potassium) is unpractical, subjected to contamination and poor sterility, and iatrogenic error from wrong determinations or extemporaneous dilutions.

From the foregoing, there is the need to have a ready to use solution, which allows adequate supplies of potassium and sodium for electrolyte maintenance and, at the same time, the maintenance of sufficient glucose levels.

The aforementioned technical problem is solved by the solutions of the present invention, which are characterized by an electrolyte concentration lower than the physiological solution, wherein a part of sodium is replaced by potassium and the chlorine ion concentration is reduced. In addition, said solutions are hypermolar than 5% glucose solutions known in the art. As already stated, the eventual limitation in physiological solution infusion (beside glucose and potassium deficiency) is that an extracellular volume expansion may occur (Holliday MA. Isotonic saline expands extracellular fluid and is inappropriate for maintenance therapy. Pediatrics. 2005 Jan;115(1):193-4; author reply 194), and hyperchloremia can be caused with a subsequent risk of acidosis (Mann C, Held U, Herzog S, Baenziger O. Impact of normal saline infusion on postoperative metabolic acidosis Paediatr Anaesth. 2009 Nov;19(11):1070-7). Accordingly, the decrease of NaCl content of the present invention, although it remains largely above that of hypotonic solutions, represents a clear improvement.

Said features provide the solutions of the present invention with improved performances. For example, glucose results ready to use for the organism, the sodium and chlorine ion concentration prevents hyponatremia without incurring in acidosis, and the presence of potassium restores the physiological concentration in child's organism.

Low osmolarity allows the peripheral administration, nevertheless, the solution can be used for central infusion as well.

The metabolic acidosis risk can be further decreased due to the presence of sodium lactate.

### Object of the invention

It is an object of the present invention a parenteral solution for pediatric use comprising per 1000 ml of solution: sodium in a concentration comprised between 90 and 120 mEq/L, chlorine in a concentration comprised between 90 and 120 mEq/L, potassium in an amount not less than 20 mEq, preferably comprised between 20 and 25 mEq/L, glucose in an amount comprised between 5% and 7.5%, and having a theoretical osmolarity lower than 750 mOsm/L.

In an embodiment of the present invention the solution comprises per 1000 ml of solution:
NaCl 100 mEq/L
K Aspartate 20 mEq/L
Glucose 50 g/L

In another embodiment of the present invention the solution comprises per 1000 ml of solution:
NaCl 80 mEq/L
Na lactate 20 mEq/L
KCI 20 mEq/L
Glucose 50 g/L

In a yet further embodiment of the present invention the solution comprises per 1000 ml of solution:
Na Lactate 100 mEq/L
KCI 20 mEq/L
Lysine chloride 80 mEq/L
Glucose 50 g/L

A further object of the present invention is the use of the above-mentioned solutions for parenteral electrolyte replacement in children, in particular in hospitalized children.

### Detailed description of the Invention

### Definitions

In the scope of the present invention, hyponatriemia means the electrolyte disturbance, wherein the plasma sodium concentration is lower than the regular one, in particular lower than 135 mEq/L.

In the scope of the present invention, maintenance means the maintenance of the electrolyte balance in the pediatric subject, which is unable to self-hydrate autonomously.

In the scope of the present invention, parenteral rehydration means the therapeutic remedy for maintaining or restoring the water, electrolyte, and metabolite pool of the organism through intravenous administration of active ingredients.

In the scope of the present invention, hydro-electrolyte maintenance solution means an aqueous electrolyte solution suitable for the sustained maintenance of solute concentrations in the aqueous environment of an organism peripheral circulation.

In the scope of the present invention, standard pediatric maintenance solution means a infusion solution, wherein 1000 ml consist of Glucose anhydrous 50.000 g, Sodium Acetate 3 H₂O 4.080 g, Sodium Chloride 1.170 g, Potassium Chloride 1.680 g, Magnesium Chloride 6 H₂O 0.508 g, Calcium Chloride.2 H₂O 0.184 g, mOsm/L 450, pH 5 (sodium 50.00 mEq/L, acetate 38.000 mEq/L, Potassium 22.500 mEq/L, Magnesium 5.0 mEq/L, Calcium 2.5 mEq/L, Chloride 50.0 mEq/L). In the scope of the present invention, Ringer lactate means a solution which is isotonic compared with blood, suitable for intravenous administration wherein 1000 ml consist of sodium ion 132 mEq (130 mmol/L), chloride ion 112 mEq (109 mmol/L), lactate 29 mEq (28 mmol/L), potassium ion 5 mEq (4 mmol/L), calcium 4 mEq (2 mmol/L).

In the solutions object of the present invention, the electrolyte concentration is lower than the physiological solution (154 mEq/L of NaCl) and a part of sodium has been replaced by potassium.

The selection of a NaCl concentration lower than the physiological solution (100 mEq/L instead of 154) arises from the fact that in the ordinary maintenance of the child hydrosaline balance an exceeding Cl supply can lead to acidosis. Practically, conventional recommendations regarding NaCl and water requirements for a healthy child, which are still performed in many hospital contexts, report very lower amounts of Na (25-30 mEq/L).

This solution shows superior performances than a simple physiological solution supplemented with 5% glucose, due to the presence of K.

Adding electrolytes to a 5% glucose solution, the osmolarity of which is about 278 mOsm/L, increases the overall osmolarity of the solution leading it to be slightly hyperosmolar having a theoretical value of about 518 mOsm/L.

Upon injection, the glucose present in the solutions is readily exploited by the organism.

Furthermore, the NaCl concentration results high enough to counterbalance hyponatremia, without achieving the levels of the physiological solution, which, on the other hand, is poor in glucose, potassium and provides an exceeding amount of chlorine for a child, leading to a consequent risk of acidosis.

The sodium lactate present is able to counteract a possible component of metabolic acidosis.

Adding K to the solution allows to maintain or restore the potassium pool. The solutions of the present invention are prepared for parenteral administration by adding suitable excipients and preservatives known to the skilled in pharmaceutical techniques.

The present invention is further illustrated by means of the following non-limiting examples.

### Examples

The solution comprising 100 mEq/L Na, 100 mEq/L Cl, 20 mEq/L K, 5% Glucose has been administered to 300 pediatric subjects, without evidence of undue events.

The solution has been presented to 300 patients under transient monitoring for maintenance during fasting or insufficient hydration due to respiratory diseases such as bronchiolitis or pneumonia, postoperative monitoring, before and after performing invasive procedures which require extended fasting such as, for example, after fracture reduction under deep sedation, gastroenteritis without remarkable dehydration, but incoercible vomiting.

The solution has been used in all the circumstances wherein maintenance hydration was required and according to the below-mentioned exclusion criteria.

The enrolled patient's age ranged within 1 month to 17 years, with an average age equal to 4.5 years. Exclusion criteria: patients able to spontaneously self-hydrate without prescription to avoid liquid assumption, patients which were prescribed to receiving physiological solution (in the cases of dehydration, distributive hypovolemia due to septic or anaphylactic shock), patients with electrolyte impairments already known, patients with hypoglycemia, patients with renal failure, patients with adrenal insufficiency, patients with cardiopathy and patients with hepatic insufficiency have been excluded.

In all treated patients, monitoring of electrolytes and glycemia before starting the infusion has been performed. A further monitoring of electrolytes and glycemia has been carried out at variable times according to the clinical context, comprised between 12 and 24 hours in 179 patients, whereas in 121 patients the monitoring of the infusion of less than 12 hours has not been performed.

In no case a significant electrolyte impairment or clinical complications eventually related to hydro-electrolyte unbalances such as convulsions, coma or arrhythmias has been observed.

## Claims

1. Parenteral solution for pediatric use comprising, per 1000 ml of sodium solution in a concentration comprised between 90 and 120 mEq/L, chlorine in a concentration comprised between 90 and 120 mEq/L, potassium in an amount comprised between 20 and 25 mEq/L , glucose in an amount comprised between 5% and 7.5%, and having a theoretical osmolarity of less than 750 mOsm/L.

2. Parenteral solution for use according to claim 1, wherein sodium is in the form of inorganic salt.

3. Parenteral solution for use according to claim 2 wherein the inorganic salt is NaCl.

4. Parenteral solution for use according to any one of claims 1-3, wherein potassium is in the form of inorganic salt.

5. Parenteral solution for use according to claim 4 wherein the inorganic salt is KCl.

6. Parenteral solution for use according to claim 1, wherein sodium is in the salt form of an organic acid.

7. Parenteral solution for use according to claim 6 wherein the organic acid is lactic or aspartic acid.

8. Parenteral solution for use according to any one of claims 1-7, wherein chlorine is in the form of lysine chloride.

9. Parenteral solution for use according to claim 1 comprising per 1000 ml of solution:
NaCl 100 mEq/L
K Aspartate 20 m Eq/L
Glucose 50 g/L

10. Parenteral solution for use according to claim 1 comprising per 1000 ml of solution:
NaCl 80 mEq/L
Na lactate 20 mEq/L
KCI 20 mEq/L
Glucose 50 g/L

11. Parenteral solution for use according to claim 1 comprising per 1000 ml of solution:
Na Lactate 100 mEq/L
KCI 20 mEq/L
Lysine chloride 80 mEq/L
Glucose 50g/L

12. Parenteral solution for use according to any claims from 1 to 11 for use as agent for electrolyte maintenance.

## Patentansprüche

1. Parenterale Lösung für die pädiatrische Verwendung, umfassend pro 1000 ml Lösung Natrium in einer Konzentration im Bereich von 90 und 120 mEq/l, Chlor in einer Konzentration im Bereich von 90 und 120 mEq/l, Kalium in einer Menge im Bereich von 20 und 25 mEq/l, Glukose in einer Menge im Bereich von 5 % und 7,5 %, und mit einer theoretischen Osmolarität von weniger als 750 mOsm/l.

2. Parenterale Lösung zur Verwendung nach Anspruch 1, wobei Natrium in Form eines anorganischen Salzes vorliegt.

3. Parenterale Lösung zur Verwendung nach Anspruch 2, wobei das anorganische Salz NaCl ist.

4. Parenterale Lösung zur Verwendung nach einem der Ansprüche 1-3, wobei Kalium in Form eines anorganischen Salzes vorliegt.

5. Parenterale Lösung zur Verwendung nach Anspruch 4, wobei das anorganische Salz KCl ist.

6. Parenterale Lösung zur Verwendung nach Anspruch 1, wobei Natrium als Salz einer organischen Säure vorliegt.

7. Parenterale Lösung zur Verwendung nach Anspruch 6, wobei die organische Säure Milchsäure oder Asparaginsäure ist.

8. Parenterale Lösung zur Verwendung nach einem der Ansprüche 1-7, wobei Chlor in Form von Lysinchlorid vorliegt.

9. Parenterale Lösung zur Verwendung nach Anspruch 1, umfassend pro 1000 ml Lösung:
NaCl 100 mEq/L
K Aspartat 20 m Eq/l
Glukose 50 g/l

10. Parenterale Lösung zur Verwendung nach Anspruch 1, umfassend pro 1000 ml Lösung:
NaCl 80 mEq/l
Na-Lactat 20 mEq/l
KCl 20 mEq/l
Glukose 50 g/L

11. Parenterale Lösung zur Verwendung nach Anspruch 1, umfassend pro 1000 ml Lösung:
Na-Lactat 100 mEq/l
KCl 20 mEq/L
Lysinchlorid 80 mEq/L
Glukose 50 g/L

12. Parenterale Lösung zur Verwendung nach einem der Ansprüche 1 bis 11 zur Verwendung als Mittel zur Elektrolyt-Aufrechterhaltung.

## Revendications

1. Solution parentérale pour son utilisation pédiatrique comprenant, pour 1000 ml d'une solution de sodium à une concentration comprise entre 90 et 120 mEq/L, du chlore à une concentration comprise entre 90 et 120 mEq/L, du potassium en une quantité comprise entre 20 et 25 mEq/L, du glucose en une quantité comprise entre 5 % et 7,5 %, et présentant une osmolarité théorique inférieure à 750 mOsm/L.

2. Solution parentérale pour son utilisation selon la revendication 1, dans laquelle le sodium est sous la forme d'un sel inorganique.

3. Solution parentérale pour son utilisation selon la revendication 2 dans laquelle le sel inorganique est NaCl.

4. Solution parentérale pour son utilisation selon l'une quelconque des revendications 1 à 3, dans laquelle le potassium est sous la forme d'un sel inorganique.

5. Solution parentérale pour son utilisation selon la revendication 4 dans laquelle le sel inorganique est KCI.

6. Solution parentérale pour son utilisation selon la revendication 1, dans laquelle le sodium est sous la forme d'un sel d'acide organique.

7. Solution parentérale pour son utilisation selon la revendication 6 dans laquelle l'acide organique est l'acide lactique ou aspartique.

8. Solution parentérale pour son utilisation selon l'une quelconque des revendications 1 à 7, dans laquelle le chlore est sous la forme de chlorure de lysine.

9. Solution parentérale pour son utilisation selon la revendication 1 comprenant pour 1000 ml de solution :
NaCl 100 mEq/L
Aspartate de K 20 mEq/L
Glucose 50 g/L.

10. Solution parentérale pour son utilisation selon la revendication 1 comprenant pour 1000 ml de solution :
NaCl 80 mEq/L
Lactate de Na 20 mEq/L
KCI 20 mEq/L
Glucose 50 g/L.

11. Solution parentérale pour son utilisation selon la revendication 1 comprenant pour 1000 ml de solution :
Lactate de Na 100 mEq/L
KCI 20 mEq/L
Chlorure de lysine 80 mEq/L
Glucose 50 g/L.

12. Solution parentérale pour son utilisation selon l'une quelconque des revendications 1 à 11 pour son utilisation comme agent de maintien d'électrolytes.
